# EUROPEAN PATENT APPLICATION

(11) **EP 4 840 446 A1**
(43) Date of publication of application: **18.11.2026**
(21) Application number: 25738382.8
(22) Date of filing: 07.01.2025
(51) Int. Cl.: A61K 31/195, A61P 25/04

(54) **USE OF FUSED TRICYCLIC GAMMA-AMINO ACID DERIVATIVE IN TREATING PATHOLOGICAL CENTRAL NERVOUS PAIN**

(30) Priority: 08.01.2024 CN 202410022737
(71) Applicant: Haisco Pharmaceutical Group Co., Ltd., Shannan, Xizang 856099 (CN)
(72) Inventor: GOU, Xiaoli, Shannan, Xizang 856000 (CN); MENG, Qingyuan, Shannan, Xizang 856000 (CN); WANG, Ju, Shannan, Xizang 856000 (CN); HUANG, Qin, Shannan, Xizang 856000 (CN); ZENG, Weifang, Shannan, Xizang 856000 (CN); LI, Fangqiong, Shannan, Xizang 856000 (CN)
(74) Representative: AWA Denmark A/S
(86) International application number: PCT/CN2025/070898
(87) International publication number: WO 2025/148851

(57) **Abstract**

The present invention relates to use of a compound represented by formula (I), or a stereoisomer, a tautomer, a deuterated form, a solvate, a prodrug, a metabolite, a pharmaceutically acceptable salt thereof, a cocrystal or a composition thereof in preparing a medicament for treating pathological central nervous pain. The present invention further relates to a method for treating pathological central nervous pain, comprising: administering an effective amount of the compound represented by formula (I), or the stereoisomer, the tautomer, the deuterated form, the solvate, the prodrug, the metabolite, the pharmaceutically acceptable salt, the cocrystal or the composition thereof.

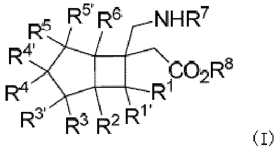

## Description

### Technical Field

The present invention relates to the use of a compound represented by formula (I), or a stereoisomer, a tautomer, a deuterated form, a solvate, a prodrug, a metabolite, a pharmaceutically acceptable salt thereof, a cocrystal or a composition thereof in the preparation of a medicament for treating central neuropathic pain.

### Background Art

Neuropathic pain is caused by damage or lesions to the nervous system. The pathogenesis of neuropathic pain is complex, involving anatomical structural changes and functional impairments. Multiple mechanisms are commonly implicated, including peripheral sensitisation, central sensitisation, dysfunction of the descending inhibitory system, activation of spinal glial cells, alterations of ion channels, etc. Central neuropathic pain is a subtype of neuropathic pain classified according to the anatomical location of the causative damage or disease, with pathological peripheral nervous pain constituting the other subtype. Common types of the central neuropathic pain comprise post-stroke pain, spinal cord injury pain, Parkinson's disease-related pain, multiple sclerosis-related pain, etc.

According to the 2013 Expert Consensus on Diagnosis and Treatment of Neuropathic Pain, calcium channel modulators (e.g., pregabalin and gabapentin), tricyclic antidepressants (e.g., amitriptyline), and serotonin-noradrenalin reuptake inhibitors (e.g., venlafaxine and duloxetine) are recommended as first-line therapeutic drugs for neuropathic pain. The 2017 Chinese Expert Consensus on Evaluation, Treatment, and Rehabilitation of Traumatic Spinal Cord Injury recommends gabapentin and pregabalin as first-line therapeutic drugs for neuropathic pain following spinal cord injury. Other drugs that can be used in the treatment include tricyclic antidepressants, opioids, local anaesthetics, etc. Pharmacotherapy constitutes the primary treatment modality for central post-stroke pain. Clinically, drugs indicated for neuropathic pain are routinely used in conventional management for post-stroke pain, including antidepressants, antiepileptics, opioids, N-methyl-D-aspartate (NMDA) receptor antagonists, antiarrhythmics, etc. For the management of early stage Parkinson's disease-related pain, nonsteroidal anti-inflammatory drugs, anxiolytics, antidepressants, etc., are primarily used as interventions. In contrast, moderate-to-advanced stage Parkinson's disease-related pain is predominantly treated with dopaminergic medications. However, due to the high prevalence of Parkinson's disease-related pain and the still insufficient understanding of its mechanisms, there is currently no unified clinical consensus regarding its clinical treatment. Clinically, for persistent pain caused by multiple sclerosis, baclofen, tizanidine, and gabapentin are first-line treatments, while diazepam and dantrolene are second-line agents. It can be seen that among the pharmacological treatments for various types of central neuropathic pain in clinical practice, calcium channel modulators such as pregabalin or gabapentin are recommended as first-line therapeutic drugs.

To date, no medicament has been approved by regulatory authorities in China for the treatment of central neuropathic pain. Internationally, pregabalin was approved by the U.S. Food and Drug Administration (FDA) in 2012 for the treatment of spinal cord injury-related neuropathic pain. In clinical trials submitted to the FDA for the evaluation of the safety and efficacy of pregabalin in the treatment of spinal cord injury-related neuropathic pain, pregabalin exerted superior efficacy over placebo in relieving pain in patients with this condition. Mirogabalin is a novel oral GABA analogue that acts as an α2δ ligand drug. It primarily targets the α2δ-1 subunit of calcium channels, featuring high binding affinity and selectivity. Moreover, mirogabalin dissociates from the α2δ-1 at a slower rate compared with pregabalin. Mirogabalin was approved by the Pharmaceuticals and Medical Devices Agency (PMDA) of Japan in 2022 for the treatment of central neuropathic pain. Although pregabalin is available on the Chinese market, its approved indications in the package insert are limited to postherpetic neuralgia and fibromyalgia, whereas mirogabalin has not yet received marketing approval in China.

In addition, although multiple medicaments with diverse mechanisms of action are currently used clinically for central neuropathic pain, the majority of patients fail to achieve adequate pain relief or show no response to the medicaments, and also suffer from severe adverse reactions. Therefore, there is an urgent clinical need for therapeutic drugs for central neuropathic pain that offer better analgesic efficacy and fewer adverse reactions.

WO 2018050046 describes a compound represented by formula (I) that has significant analgesic effects,

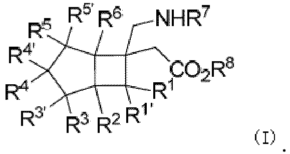

### Summary of the Invention

The present invention provides the use of a compound represented by formula (I), or a stereoisomer, a tautomer, a deuterated form, a solvate, a prodrug, a metabolite, a pharmaceutically acceptable salt thereof, a cocrystal or a composition thereof in the preparation of a medicament for treating central neuropathic pain, 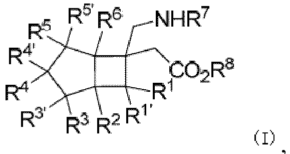
wherein R¹ and R⁴ are combined to form -(CR⁹R^{9'})n- or -CR⁹=CR^{9'}-;
R^{1'}, R², R³, R^{3'}, R^{4'}, R⁵, R^{5'}, R⁶, R⁹ or R^{9'} is each independently selected from H, F, Cl, Br, I, hydroxyl, amino, carboxyl, a carboxylate ester group, amido, cyano, C₁₋₆ alkyl, C₁₋₆ alkoxy, C₁₋₆ sulphanyl, C₂₋₆ alkenyl, C₂₋₆ alkynyl, 3- to 6-membered carbocyclyl or 3- to 6-membered heterocyclyl, wherein the alkyl, alkoxy, sulphanyl, alkenyl, alkynyl, carbocyclyl or heterocyclyl is optionally further substituted with 0-6 groups selected from F, Cl, Br, I, hydroxyl, amino, carboxyl, C₁₋₆ alkyl, 3- to 6-membered carbocyclyl or 3- to 6-membered heterocyclyl, and the heterocyclyl contains 1-2 heteroatoms selected from N, O or S; n is selected from 1, 2 or 3;
the pharmaceutically acceptable salt is selected from benzenesulphonate, p-toluenesulphonate or methanesulphonate.

Further, the compound represented by general formula (I) has a structure selected from one of the structures shown in Table S-1:

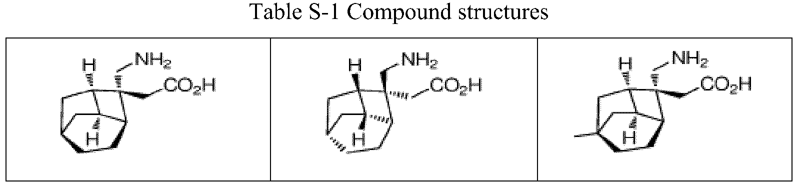

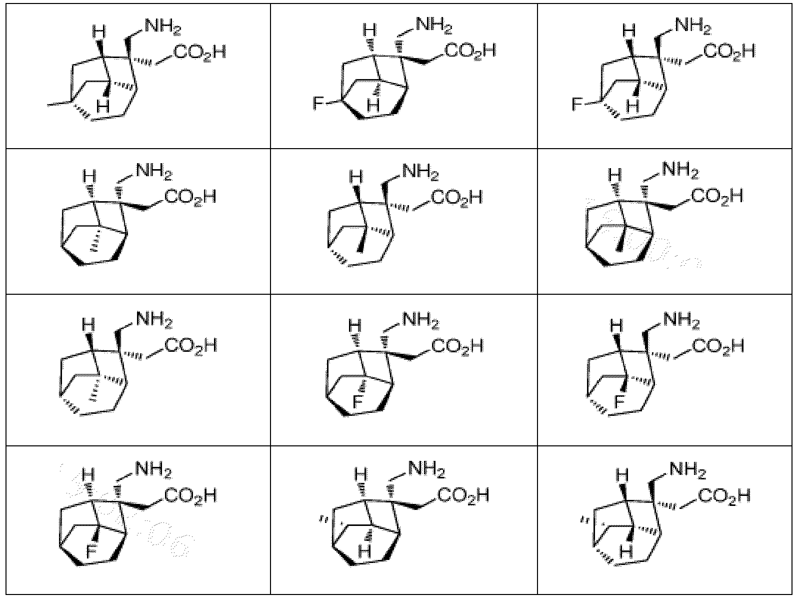

In some embodiments, the compound represented by general formula (I) has a structure of a compound represented by formula (II): 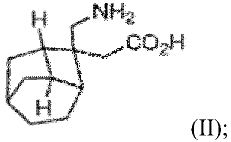
in some embodiments, the compound represented by general formula (I) has a structure of a compound represented by formula (III): 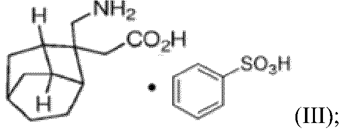
in some embodiments, the compound represented by general formula (I) has a structure of a compound represented by formula (IV): 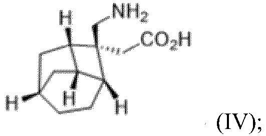
in some embodiments, the compound represented by general formula (I) has a structure of a compound represented by formula (V):

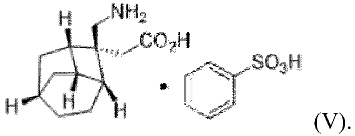

In some embodiments, the use is administering to a mammal an effective amount of the compound represented by formula (IV), or the stereoisomer, the tautomer, the deuterated form, the solvate, the prodrug, the metabolite, the pharmaceutically acceptable salt, the cocrystal or the composition thereof.

The present invention relates to central neuropathic pain selected from at least one of post-stroke pain, spinal cord injury pain, Parkinson's disease-related pain, multiple sclerosis-related pain, and other pathological pain caused by damage to the central nervous system.

In some embodiments of the present invention which relate to the use in the medicament for treating central neuropathic pain and the treatment method, an effective amount of the compound represented by formula (IV), or the stereoisomer, the tautomer, the deuterated form, the solvate, the prodrug, the metabolite, the pharmaceutically acceptable salt, the cocrystal or the composition thereof is administered to a mammal, and in terms of the compound of structural formula (IV), the amount of the compound is 1-100 mg/day, preferably 5-20 mg/day.

In some embodiments of the present invention which relate to the use in the medicament for treating central neuropathic pain and the method for treating central neuropathic pain, the compound represented by formula (IV), or the stereoisomer, the tautomer, the deuterated form, the solvate, the prodrug, the metabolite, the pharmaceutically acceptable salt, the cocrystal or the composition thereof is administered via a route selected from injection, drip, transdermal route, sublingual route, parenteral route, intraperitoneal route, rectal route, buccal route, nasal drip, inhalation, local delivery, subcutaneous route, intra-adipose route, intra-articular route, intraperitoneal route or intrathecal route.

In some embodiments of the present invention which relate to the use in the medicament for treating central neuropathic pain and the method for treating central neuropathic pain, the compound represented by formula (IV), or the stereoisomer, the tautomer, the deuterated form, the solvate, the prodrug, the metabolite, the pharmaceutically acceptable salt, the cocrystal or the composition thereof is administered via a route selected from intravenous injection, intravenous drip, intra-arterial injection, intramuscular injection, subcutaneous injection, intra-articular injection, intraperitoneal injection, intrathecal injection or nasal drip.

Unless stated to the contrary, the terms used in both the description and the claims have the following meanings:
The "pharmaceutical composition" represents a mixture of one or more compounds described herein or a physiologically/pharmaceutically acceptable salt thereof or a stereoisomer, a solvate, a pharmaceutically acceptable salt or a cocrystal thereof and other components comprising physiologically/pharmaceutically acceptable carriers and/or excipients.

The "stereoisomer" refers to an isomer produced as a result of different spatial arrangement of atoms in molecules, including cis-trans isomers, enantiomers and conformational isomers.

The "effective amount" refers to an amount that causes a physiological or medical response in a tissue, system or subject and is a desirable amount, including the amount of a compound that is, when administered to a subject, sufficient to prevent occurrence of one or more symptoms of the disease or condition to be treated or to reduce the symptom(s) to a certain degree.

The "solvate" refers to the compounds of the present invention or the salts thereof, and also includes stoichiometric or non-stoichiometric solvents bound by intermolecular non-covalent forces. When the solvent is water, the solvate is a hydrate.

### Brief Description of the Drawings

Figure 1 shows the mechanical pain threshold of animals in each group before model establishment (baseline) in Test example 1 of the present invention.
Figure 2 shows the pre-administration (0-hour) mechanical pain threshold of animals in each group in Test example 1 of the present invention.
Figure 3 shows the analgesic effects of compound (V) at 2 hours post-administration in a mouse model of spinal cord injury-induced central neuropathic pain in Test example 1 of the present invention.
Figure 4 shows the analgesic effect of compound (V) at 4 hours post-administration in a mouse model of spinal cord injury-induced central neuropathic pain in Test example 1 of the present invention.
Figure 5 shows the analgesic effect of compound (V) at 6 hours post-administration in a mouse model of spinal cord injury-induced central neuropathic pain in Test example 1 of the present invention.
Figure 6 shows the area under the curve (AUC) of mechanical pain threshold changes of animals in each group in Test example 1 of the present invention.
Figure 7 shows the overall design diagram of the clinical study of the present invention.

### Detailed Description of Embodiments

The technical solutions of the present invention will be described in detail below in conjunction with the drawings and examples, but the scope of protection of the present invention includes but is not limited thereto.

### Synthesis example:

**Compounds represented by formulas (IV) and (V)** were prepared according to the methods described in WO 2018050046:

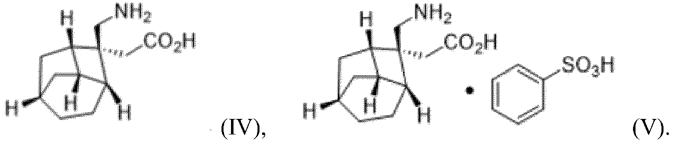

### Biological test examples

Test example 1: Study on analgesic effects of compound (V) in a mouse model of spinal cord injury-induced central neuropathic pain
Female C57BL/6J mice obtained from Zhejiang Vital River Laboratory Animal Technology Co., Ltd. were acclimated for one week prior to the start of the experiment. The specific experimental procedure was as follows:
(1) Three days before the experiment, each mouse was stroked for 5 minutes daily to habituate the animals to the experimenter. Thereafter, the mice were placed on a metal stand for pain testing for 60 minutes of acclimation.
(2) Baseline mechanical pain thresholds were measured in all experimental animals (using the Ascending method). Each animal was tested twice with an interval of at least 5 minutes between measurements, and the average value was calculated. Animals were subjected to 1st grouping (Sham group and model group) using the random function in Excel based on animal numbers.
(3) The Sham group underwent a sham surgery, while the remaining animals received a spinal cord partial transection injury to establish spinal cord injury (SCI) models. The day of modelling was designated as Day 0.
(4) On day 25 (Day 25) post-modelling, all mice were assessed using the BBB score for spinal cord injury. Animals failing to meet the modelling criteria (qualified range defined as mean ± standard deviation of BBB scores) were excluded.
(5) Pre-administration mechanical pain testing: Mechanical pain thresholds of the mice were measured on day 26 (Day 26) post-modelling, which served as the 0-hour (pre-administration) data.
(6) Pre-administration animal grouping: Based on the pre-administration mechanical pain threshold (MPT), mice with no significant reduction in pain threshold (MPT > 0.4 g) were excluded. The remaining model animals were further divided equally into experimental groups according to different pain threshold levels using the random function in Excel.
(7) Drug administration and post-administration mechanical pain threshold testing: On day 28 (Day 28) post-modelling, the compound (V) formulated in vehicle at different doses (calculated as free base), or vehicle alone (0.5% methylcellulose), was administered by gavage. Mechanical pain thresholds of the mice were measured at 2, 4, and 6 hours after a single administration.

The method for establishing a mouse model of spinal cord injury-induced central neuropathic pain was as follows:
(1) The air pump of the respiratory anaesthesia machine was turned on, and the oxygen flow adjustment knob was rotated counterclockwise to adjust to 0.8 L/min; the animal was placed into the induction chamber, and the gas switch was turned to the "Chamber" position, allowing the anaesthetic gas to flow into the induction chamber; the dial lock button was pressed, and the vaporiser dial was rotated to adjust the anaesthetic gas concentration to 2 L/min. After induction anaesthesia was completed, the vaporiser dial was rotated to adjust the anaesthetic gas concentration to 1 L/min, and the gas switch was turned to the "Mask" position, so that the gas flowed to the anaesthesia mask. The animal was removed from the anaesthesia induction chamber, and its head/nose was placed into the anaesthesia mask. After the animal was confirmed to be under adequate anaesthesia, the subsequent experimental procedures could be performed.
(2) The surgical instruments were sterilised, the dorsal hair was removed, and the skin was disinfected with iodophor.
(3) The T9 vertebra was identified along the 13th rib on the dorsal side.
(4) A 1-2 cm midline skin incision was made on the back, centred on the spinous process of the T9 vertebra.
(5) The subcutaneous tissue and muscles were bluntly dissected to expose the spinous process and lamina of the T9 vertebra.
(6) The spinous process of T9 was clamped and gently lifted upward and to the left to open the intervertebral space. The right intervertebral space of T9 was used as the surgical approach. The ligamentum flavum was incised, and the junctions between the T9 transverse process and its two adjacent transverse processes were cut sequentially. The T9 spinous process was lifted upward as flatly as possible from the abdomen using the middle finger of the left hand to enlarge the intervertebral space.
(7) Ophthalmic surgical blade was held in the right hand with the blade edge facing the side to be transected. The blade tip was inserted into the intervertebral space parallel to the T9 spinous process, and then the blade was cut vertically downward until reaching the vertebra. Simultaneously, the blade handle was tilted at a certain angle toward the non-transected side. Twitching of the hindlimb on the transected side was considered as a sign of successful model preparation.
(8) The muscle and skin layers were sutured sequentially with sutures, and the surgical site was disinfected.
(9) For the Sham group, only steps (1)-(5) were performed, after which the muscle and skin layers were sutured sequentially with sutures, and the surgical site was disinfected.
(10) After the surgery was completed, the vaporiser dial was rotated, the isoflurane valve and oxygen valve were closed, the oxygen flow adjustment knob was rotated clockwise, the air pump was turned off, and the anaesthesia induction chamber was cleaned.

BBB scoring method for spinal cord injury is as described in the following table:

| **Score** | **Feature** | **Description** |
|---|---|---|
| 0 | No observable movement of the hindlimbs | |
| 1 | Slight movement of one or two joints, usually hip and(or) knee | Slight: ≤ 5% of joint range of motion |
| 2 | Extensive movement of one joint, or extensive movement of one joint and slight movement of the other | Extensive: > 50% of joint range of motion |
| 3 | Extensive movement of two joints | The two joints are usually hip and ankle joints |
| 4 | Slight movement of all three joints of the hindlimbs | The three joints are hip, ankle, and ankle joints |
| 5 | Slight movement of two joints and extensive movement of the third joint | The third joint is usually the ankle joint |
| 6 | Extensive movement of two joints and slight movement of the third joint | |
| 7 | Extensive movement of all three joints of the hindlimbs | |
| 8 | Sweeping without weight bearing or paw placement in a non-weight-bearing position (plantar support of the paw without weight bearing) | |
| 9 | Plantar support of the paw with weight bearing only in the support stage, or occasional/frequent/consistent dorsal stepping with weight bearing | Weight bearing: hindlimb extensor contraction occurs either when the paw is in a plantar weight-bearing position, or only when the posterior trunk is elevated |
| 10 | Plantar stepping with occasional weight bearing and no forelimb-hindlimb coordination | Occasional: > 5% and ≤ 50% |
| 11 | Plantar stepping with frequent to consistent weight bearing and no forelimb-hindlimb coordination | Frequent: 51%-95% of observation period consistent: 95%-100% of observation period |
| 12 | Plantar stepping with frequent to consistent weight bearing and occasional forelimb-hindlimb coordination | 6%-50% coordinated movement |
| 13 | Plantar stepping with frequent to consistent weight bearing and frequent forelimb-hindlimb coordination | 51%-95% coordinated movement |
| 14 | Consistent plantar stepping and forelimb-hindlimb coordination, and the predominant paw position is rotated at initial contact with the ground and lift off; frequent plantar stepping, consistent hindlimb coordination, and occasional dorsal stepping | Rotation: internal or external rotation of the paw at initial contact with the ground and lift off |
| 15 | Consistent plantar stepping and forelimb-hindlimb coordination during gait; no toe clearance or occasional toe clearance during forward limb advancement; the predominant paw position is parallel to the body at initial contact with the ground | Parallel: hindpaw parallel to the trunk at initial contact with the ground or lift off toe clearance: assessed by listening for toe drag sound, i.e., toe extension is present when there is no toe drag sound during paw placement |
| 16 | Consistent plantar stepping and forelimb-hindlimb coordination during gait; toe clearance occurs frequently; the predominant paw position is parallel to the body at initial contact with the ground and rotated at lift off | Frequent toe clearance: no toe drag sound in half or more of paw placements |
| 17 | Consistent plantar stepping and forelimb-hindlimb coordination during gait; toe clearance occurs frequently; the predominant paw position is parallel to the body at initial contact with the ground and lift off | |
| 18 | Consistent plantar stepping and forelimb-hindlimb coordination during gait; toe clearance occurs consistently; the predominant paw position is parallel to the body at initial contact with the ground and lift off | Consistent toe clearance: ≤ 4 toe drag sounds during a 4-minute observation period |
| 19 | Consistent plantar stepping and forelimb-hindlimb coordination during gait; toe clearance occurs consistently; the predominant paw position is parallel to the body at initial contact with the ground and lift off; and the animal presents a downward tail throughout part or all of the observation period | |
| 20 | Consistent plantar stepping and forelimb-hindlimb coordination during gait; toe clearance occurs consistently; the predominant paw position is parallel to the body at initial contact with the ground and lift off; and the animal presents consistent elevation of the tail and trunk instability throughout part or all of the observation period | Elevation of the tail: not touching the ground trunk instability: swaying, tilting or slipping occurs with lateral shift of the centre of gravity during rapid movement |
| 21 | Consistent plantar stepping and forelimb-hindlimb coordination during gait; toe clearance occurs consistently; the predominant paw position is parallel to the body at initial contact with the ground and lift off; and the animal presents consistent elevation of the tail and consistent trunk stability throughout part or all of the observation period | Consistent trunk stability: no slipping occurs, and the pelvic ring remains aligned with the tail during locomotion |

The method for measuring mechanical pain threshold (MPT) (the Ascending method) was as follows:
(1) The experimental animal was placed on a metal mesh stand for mechanical pain testing and allowed to acclimate for 1 hour. Testing was initiated only after the animal remained relatively calm without rearing or exploratory behaviour.
(2) Once the animal was calm, a Von Frey filament (North Coast, Cat. No. NC12775-99) was slowly and gently applied vertically to the plantar surface of the hindlimb to be tested, causing the filament to bend. The stimulus was maintained for 2-3 seconds, and paw withdrawal response was observed. The filaments were applied to the test animal in ascending order of gram force. Each filament strength was applied for five consecutive stimuli, with an inter-stimulus interval of at least 10 seconds. If fewer than three positive responses were observed, the next higher-force filament was used, and the above-described procedure was repeated. The first filament that induced three or more positive responses was recorded as the pain threshold for that animal (each animal was tested twice, and the average value was calculated).
(3) A response was marked as positive (×) if the animal showed paw lifting, evasion, or paw licking due to the stimulus; paw withdrawal caused by body movement was not counted; a lack of the above behavioural responses was marked as negative (o).
(4) Filament forces: 0.16 g, 0.4 g, 0.6 g, 1.0 g, 1.4 g and 2.0 g, with a cut-off value of 2.0 g.

After the testing was completed, the area under the curve (AUC) was calculated based on the mechanical pain threshold (MPT) at each time point before and after administration using the following formula: AUC = MPT₀ₕ + 2×MPT₂ₕ + 2×MPT₄ₕ + MPT₆ₕ. Statistical analysis and graph plotting were performed using GraphPad Prism 8 software.

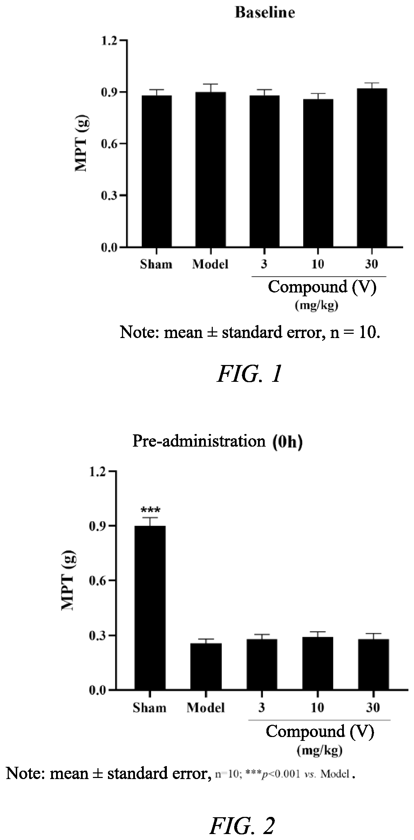

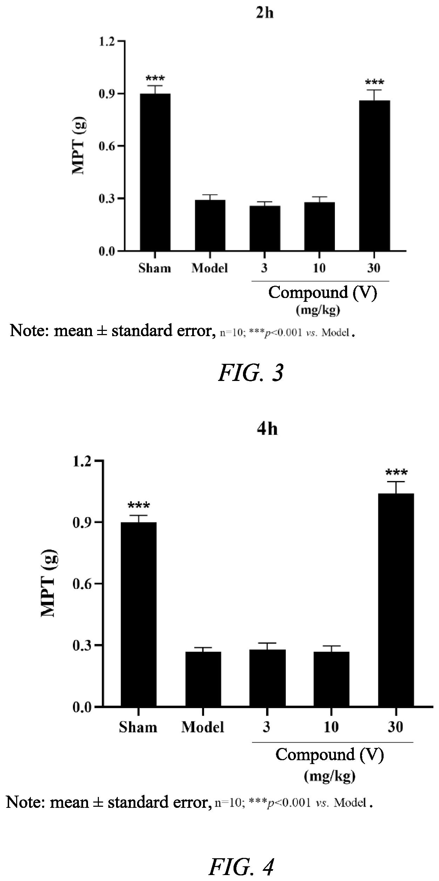

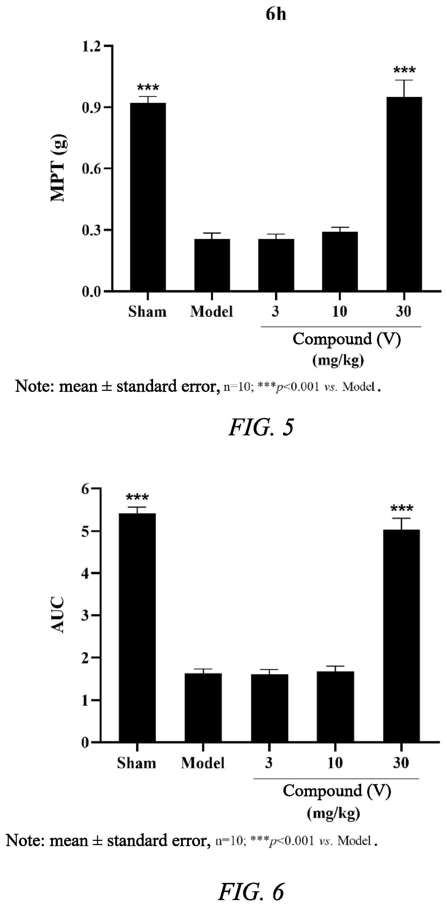

The results are shown in Tables 1 and 2 and Figures 1-6. At a dose of 30 mg/kg, compound (V) exhibited extremely significant analgesic effects, and the mechanical pain threshold of this group was comparable to that of the Sham group.

Table 1 summarises the mechanical pain thresholds (mean ± standard error) of animals in each group in Test example 1 of the present invention.

**Table 1**

| **Group** | **Mechanical pain threshold (g)** | | | | |
|---|---|---|---|---|---|
| | **Baseline** | **Pre-administration** | **Post-administration** | | |
| | | **0 h** | **2 h** | **4 h** | **6 h** |
| Sham | 0.88 ± 0.03 | 0.90 ± 0.04 | 0.90 ± 0.04 | 0.90 ± 0.03 | 0.92 ± 0.03 |
| Model | 0.90 ± 0.04 | 0.26 ± 0.02 | 0.29 ± 0.03 | 0.27 ± 0.02 | 0.26 ± 0.03 |
| Compound (V), 3 mg/kg | 0.88 ± 0.03 | 0.28 ± 0.03 | 0.26 ± 0.02 | 0.28 ± 0.03 | 0.26 ± 0.02 |
| Compound (V), 10 mg/kg | 0.86 ± 0.03 | 0.29 ± 0.03 | 0.28 ± 0.03 | 0.27 ± 0.03 | 0.29 ± 0.02 |
| Compound (V), 30 mg/kg | 0.92 ± 0.03 | 0.28 ± 0.03 | 0.86 ± 0.06 | 1.04 ± 0.06 | 0.95 ± 0.08 |

Table 2 summarises the area under the curve (mean ± standard error) of mechanical pain threshold changes of animals in each group in Test example 1 of the present invention.

**Table 2**

| **Group** | **Area under the curve (AUC) of mechanical pain threshold changes** |
|---|---|
| Sham | 5.42 ± 0.14 |
| Model | 1.63 ± 0.10 |
| Compound (V), 3 mg/kg | 1.61 ± 0.11 |
| Compound (V), 10 mg/kg | 1.68 ± 0.12 |
| Compound (V), 30 mg/kg | 5.03 ± 0.27 |

**Clinical trial: Multicentre, randomised, double-blind, parallel, placebo-controlled phase III clinical trial to evaluate the efficacy and safety of compound (V) capsules in Chinese patients with moderate-to-severe central neuropathic pain.**

**Trial duration:** The total duration of participation for each subject is approximately 56 weeks, consisting of: 2-week screening period (14 days prior to the run-in period), 1-week run-in period [including baseline (D0)], 12-week double-blind treatment period, 40-week open-label extension treatment period, and 1-week safety follow-up period.

### Trial objectives

### Primary objective

To evaluate the efficacy of compound (V) capsules (hereinafter referred to as compound (V)) compared with placebo in the treatment of moderate-to-severe central neuropathic pain.

### Secondary objectives

To evaluate the safety of compound (V) compared with placebo in the treatment of moderate-to-severe central neuropathic pain.

To evaluate the long-term safety and efficacy of compound (V) in the treatment of moderate-to-severe central neuropathic pain (52 weeks).

To evaluate the pharmacokinetic (PK) profile of compound (V) in Chinese patients with moderate-to-severe central neuropathic pain.

### Overall trial design

This trial employed a multicentre, randomised, double-blind, placebo-controlled, parallel-group design.

The trial consisted of a 2-week screening period (14 days prior to the run-in period), a 1-week run-in period [including baseline (D0)], a 12-week double-blind treatment period, a 40-week open-label extension treatment period, and a 1-week safety follow-up period.

Subjects entered the screening period after signing the informed consent form (ICF). Subjects who completed the protocol-required screening assessments during the screening period and met all inclusion criteria and none of the exclusion criteria entered the placebo run-in period, during which they received placebo once every morning and evening (1 capsule per dose, BID) for 1 consecutive week. Subjects underwent randomisation eligibility assessment at baseline (D0). Eligible subjects were randomised in a 1:1 ratio to either the compound (V) group or the placebo group. Stratified randomisation was performed based on baseline ADPS score (< 6 and ≥ 6) and disease type (spinal cord lesion-related neuropathic pain, post-stroke central neuropathic pain, Parkinson's disease-related pain, and multiple sclerosis-related pain).

Medication treatment period: The medication treatment period consisted of two phases: a 12-week double-blind treatment period and a 40-week open-label extension treatment period.

Double-blind treatment period: Subjects who met the randomisation criteria received oral compound (V) capsules or placebo twice daily (BID). Randomisation eligibility assessment was performed at baseline (D0). Eligible subjects began receiving the trial treatment (at a dose of 20 mg) in the evening of the randomisation day (D0) at Visit V3, and starting from the next day, the dose was administered at 20 mg BID. At Visit V4 (end of the second week of medication treatment), tolerability and efficacy response of the subjects were assessed. If the subject's PGIC score was "very much improved," "much improved," or "minimally improved," the 20 mg BID dose was maintained. If the subject's PGIC score was "no change," "minimally worse," "much worse," or "very much worse," and tolerability was good (no occurrence of Grade 3 or higher adverse drug reactions (ADRs)), the dose was adjusted to 40 mg BID. At Visit V5 (end of the fourth week), subject tolerability was assessed. For subjects whose dose had been adjusted to 40 mg BID, if the dose was not tolerated (severe dizziness, somnolence, or occurrence of Grade 3 or higher ADRs), the dose could be reduced back to 20 mg BID. No further dose adjustments were permitted from Visit V5 to Visit V7 (end of the double-blind treatment period), i.e., from the start of Week 5 through the end of Week 12.

Open-label extension treatment period: Subjects who completed the 12-week double-blind treatment period without experiencing any major safety issues could voluntarily enter the 40-week open-label extension treatment period. For subjects who voluntarily entered the open-label extension treatment period, open-label extension trial treatment began on the evening of Visit V7 (at a dose of compound (V) 20 mg). Starting the next day, subjects received compound (V) at 40 mg/day (20 mg BID). During the open-label extension treatment period, subjects could have their dose adjusted under the guidance of the investigator based on their own efficacy response and tolerability. Specifically, for subjects receiving 20 mg BID, if the efficacy response was insufficient, the dose could be increased to 40 mg BID. Thereafter, the investigator could decide whether to reduce the dose back to 20 mg BID based on the subject's tolerability and pain relief status. After completing 52 weeks of treatment, subjects attended a safety follow-up visit on Day 7 following the last dose of the investigational product.

For subjects who did not wish to participate in the open-label extension treatment period, after completing the 12-week double-blind treatment period, they attended a safety follow-up visit on Day 7 following the last dose of the investigational product.

Subjects who did not complete the trial treatment were required to complete an early termination visit.

Rescue medications were not permitted within one week prior to the end of the run-in period or the end of the double-blind treatment period.

Rescue medication administration method: One tablet per administration, every 4-6 hours, with no more than 4 tablets per day, and not to be taken consecutively for more than 5 days.

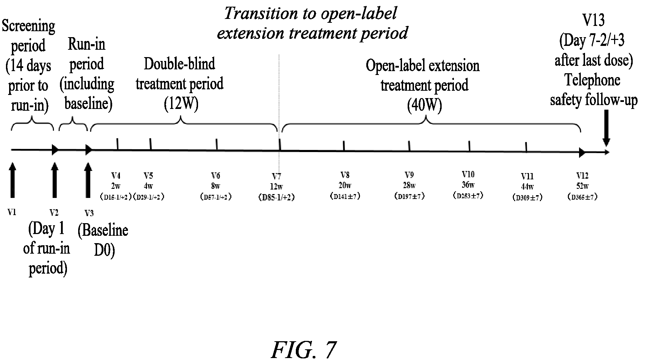

The overall trial design is as shown in Figure 7.

### Pharmacokinetic blood sampling

PK blood samples were collected from all subjects. PK blood sample collection time points included: within 30 minutes before the morning dose at Visit V4 (D15-1/+2); within 30 minutes before the morning dose at Visit V5 (D29-1/+2); at Visit V6 (D57-1/+2): within 30 minutes before the morning dose, between 30 minutes and 2 hours after the morning dose, and between 4 and 12 hours after the morning dose (before the evening dose). Approximately 3 mL of whole blood was collected at each time point. For all visits involving PK blood sampling, the subject's blood collection time and dosing time were recorded. At Visits V4, V5 and V6, the breakfast time on the day of blood sampling was also recorded.

### Number of subjects

A total of 204 subjects were enrolled for each disease type, with 102 subjects in the compound (V) group and 102 subjects in the placebo group. Enrolment could be stopped once the required number of subjects was achieved for two disease types.

### Inclusion criteria (subjects who meet all of the following criteria are eligible for enrolment in this trial)

1) Voluntarily sign the informed consent form;
2) Able to read and complete questionnaires;
3) Male or female patients aged ≥ 18 years;
4) Subjects have a relevant medical history and symptoms of central neuropathic pain, including spinal cord lesion-related neuropathic pain, central post-stroke pain, Parkinson's disease-related pain, and multiple sclerosis-related pain, and must meet the following criteria:
   a) pain duration of ≥ 3 months;
   b) meeting the characteristics of pathological neuropathic pain: Douleur Neuropathique 4 (DN4) scale score ≥ 4;
      at the screening visit, the patient's self-assessed average pain Visual Analogue Scale (VAS) score over the past 24 hours ≥ 40 mm.

### Exclusion criteria (subjects who meet any of the following criteria are not eligible for enrolment in this trial)

1) Presence of other pain that, in the investigator's assessment, may affect the evaluation of central neuropathic pain, including but not limited to:
   a) pain caused by nerve root compression (e.g., intervertebral disc herniation/protrusion, spinal stenosis, vertebral degeneration/hyperplasia) within 12 months prior to screening, except in patients with confirmed absence of nerve compression on imaging after treatment;
   b) pain caused by gouty arthritis, rheumatoid arthritis, osteoarthritis, soft tissue injury, etc., within 6 months prior to screening;
   c) pain caused by peripheral neuropathy or acquired peripheral neuropathy due to other causes within 6 months prior to screening;
   d) previous history of diabetic peripheral neuropathic pain;
   e) previous history of postherpetic neuralgia (except cured patients: those whose herpes zoster had resolved at screening and who had no postherpetic pain, skin lesions, or related symptoms/signs within 6 months prior to screening);
2) Unstable spinal cord lesions or stroke, with anticipated need for surgical therapy;
3) Presence of chronic systemic diseases that, in the investigator's assessment, may affect the subject's participation in the trial, including but not limited to:
   a) severe cardiopulmonary diseases such as unstable angina pectoris, acute myocardial infarction within 6 months, severe arrhythmias, World Health Organization (WHO) cardiac functional classification III-IV at screening, poorly controlled hypertension despite active treatment (systolic blood pressure > 160 mmHg or diastolic blood pressure > 100 mmHg at screening); recurrent asthma attacks;
   b) chronic digestive system diseases such as liver fibrosis or other liver diseases, recurrent dyspepsia or diarrhoea, or peptic ulcers;
   c) presence of other neurological or psychiatric diseases that, in the investigator's judgment, may affect self-assessment, including but not limited to moderate-to-severe cognitive impairment, somatoform disorders, epilepsy, recurrent dizziness, headache, or memory impairment;
4) Diabetic patients with poor glycaemic control (fasting blood glucose ≥ 11.1 mmol/L at screening);
5) Known history of drug abuse or known history of alcohol abuse within 1 year prior to screening [alcohol consumption exceeding 14 units per week, where 1 unit is equivalent to 360 mL of beer, 45 mL of 40% spirits, or 150 mL of wine];
6) Prior use of pregabalin ≥ 300 mg/day or gabapentin ≥ 1200 mg/day with reported lack of clinical efficacy;
7) Significant abnormalities in haematology , liver, or kidney function, meeting any of the following clinical laboratory findings:
   a) haematology : white blood cell count < 3 × 10⁹/L, neutrophils < 1.5 × 10⁹/L, platelets < 75 × 10⁹/L, or haemoglobin < 90 g/L;
   b) liver function: alanine aminotransferase (ALT) or aspartate aminotransferase (AST) > 2.5 × upper limit of normal (ULN); or total bilirubin (TBIL) > 1.5 × ULN;
   c) estimated glomerular filtration rate (eGFR) < 60 mL/min/1.73 m² (calculated using the simplified MDRD formula);
   d) creatine kinase > 2.0 × ULN;
8) History of malignancy: subjects with basal cell carcinoma, localized squamous cell carcinoma of the skin, or carcinoma in situ of cervix may be enrolled in this trial if curative treatment was completed at least 12 months prior to signing the informed consent form; subjects with other malignancies may be enrolled in this trial if curative treatment was completed at least 5 years prior to signing the informed consent form;
9) Positive for hepatitis B surface antigen (HBsAg) or hepatitis C virus antibody (HCV Ab) at screening [requiring further confirmation by hepatitis B virus deoxyribonucleic acid (HBV DNA) titre testing or hepatitis C virus ribonucleic acid (HCV RNA) testing; subjects with levels above the lower limit of detection will be excluded]; positive for human immunodeficiency virus antibody (HIV Ab); positive for Treponema pallidum antibody (TP Ab) (requiring further treponemal titre testing; if positive, exclusion is required);
10) Subjects who received prohibited medications prior to screening, or modified restricted concomitant medications within 30 days prior to screening (excluding those who discontinued restricted medications for more than 5 half-lives prior to screening and intended not to use them throughout the trial period); subjects who have taken prohibited medications prior to screening must discontinue such medications for at least 5 half-lives (half-lives shall be subject to the instructions) before the screening visit to be eligible for screening, and shall refrain from using these medications throughout the entire trial treatment period;
11) Known history of hypersensitivity to pregabalin, the investigational product, acetaminophen, other drugs with similar chemical structures, or excipients;
12) History of suicidal behaviour or suicidal ideation;
13) Pregnant women, women planning to become pregnant during the trial period, or breastfeeding women; subjects who are unwilling to use reliable contraceptive measures (including condoms, spermicides, intrauterine devices, etc.) from the time of signing the ICF until 28 days after the last dose of the investigational product, or who plan to use progesterone-containing contraceptives during this period;
14) Subjects engaged in high-altitude work, motor vehicle driving, or other hazardous mechanical operations during the trial period;
15) Participation in any other clinical trial within 30 days prior to screening;
16) Any other condition deemed by the investigator to make the subject unsuitable for participation in the trial.

### Randomisation criteria (subjects must meet any of the following criteria after completion of the run-in period, prior to entry into the double-blind treatment period)

1) Meet all inclusion criteria and none of the exclusion criteria;
2) Pain VAS score ≥ 40 mm at both the run-in period and the baseline visit;
3) Daily pain Numerical Rating Scale (NRS) score ≥ 4 during the 1-week run-in period, and must have completed at least 4 days of pain NRS assessments;
4) Subjects with low variability in daily NRS pain scores during the 1-week run-in period (difference between any two NRS pain scores < 4 points) and minimal placebo effect (VAS score reduction from V1 to V3 < 30%);
5) Investigational product compliance during the run-in period between 80%-120%.

### Trial endpoints

### Primary endpoints

1) To compare the change from baseline in ADPS at Week 12 between compound (V) and placebo in the spinal cord lesion population;
2) To compare the change from baseline in ADPS at Week 12 between compound (V) and placebo in the overall population;
3) To compare the change from baseline in ADPS at Week 12 between compound (V) and placebo in the post-stroke central neuropathic pain population.

### Secondary endpoints

1) To evaluate the ADPS responder rate (proportion of subjects with a ≥ 30% and ≥ 50% reduction from baseline in weekly ADPS) at Week 12 between compound (V) and placebo, by disease type and in the overall population;
2) To compare the weekly ADPS change from baseline from Week 1 to Week 12 between compound (V) and placebo, by disease type and in the overall population;
3) To evaluate the change from baseline in VAS score at Week 12 between compound (V) and placebo, by disease type and in the overall population;
4) To evaluate the change from baseline in Short-Form McGill Pain Questionnaire (SF-MPQ) score at Week 12 between compound (V) and placebo, by disease type and in the overall population;
5) To evaluate the change from baseline in Average Daily Sleep Interference Scale (ADSIS) score at Week 12 between compound (V) and placebo, by disease type and in the overall population;
6) To evaluate the impact on quality of life of subjects as measured by the EuroQol Five-Dimension Five-Level Scale (EQ-5D-5L) at Week 12 between compound (V) and placebo, by disease type and in the overall population;
7) To evaluate the change from baseline in Hospital Anxiety and Depression Scale (HADS) score at Week 12 between compound (V) and placebo, by disease type and in the overall population;
8) To evaluate the change from baseline in POMS-C score at Week 12 between compound (V) and placebo, by disease type and in the overall population;
9) To evaluate the Patient Global Impression of Change (PGIC) at Week 12, by disease type and in the overall population;
10) To evaluate the change from baseline in SF-MPQ (including VAS) at Week 52, by disease type and in the overall population;
11) To evaluate the change from baseline in EQ-5D-5L score at Week 52, by disease type and in the overall population.

### Pharmacokinetic indicators:

Plasma concentrations of compound (V) at specified time points (V4, V5, and V6) within 12 weeks of oral administration in patients with central neuropathic pain.

### Safety indicators:

To compare the AEs, laboratory tests, physical examinations, vital signs, and 12-lead ECG between compound (V) and placebo over 12 weeks of oral administration, and to evaluate the safety and tolerability of compound (V);
to evaluate the safety and tolerability of compound (V) over 52 weeks of oral administration.

### Trial conclusion:

The above clinical trial results demonstrate that compound (V) has good efficacy and safety for the treatment of moderate-to-severe central neuropathic pain.

## Claims

1. Use of a compound represented by formula (I), or a stereoisomer, a tautomer, a deuterated form, a solvate, a prodrug, a metabolite, a pharmaceutically acceptable salt thereof, a cocrystal or a composition thereof in the preparation of a medicament for treating central neuropathic pain, **characterized in that** an effective amount of the compound represented by formula (I), or the stereoisomer, the tautomer, the deuterated form, the solvate, the prodrug, the metabolite, the pharmaceutically acceptable salt, the cocrystal or the composition thereof is administered to a mammal;
the formula (I) has a structure as follows: 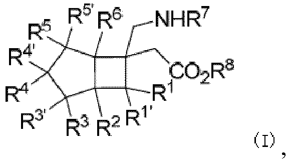
wherein R¹ and R⁴ are combined to form -(CR⁹R^{9'})n- or -CR⁹=CR^{9'}-;
R^{1'}, R², R³, R^{3'}, R^{4'}, R⁵, R^{5'}, R⁶, R⁹ or R^{9'} is each independently selected from H, F, Cl, Br, I, hydroxyl, amino, carboxyl, a carboxylate ester group, amido, cyano, C₁₋₆ alkyl, C₁₋₆ alkoxy, C₁₋₆ sulphanyl, C₂₋₆ alkenyl, C₂₋₆ alkynyl, 3- to 6-membered carbocyclyl or 3- to 6-membered heterocyclyl, wherein the alkyl, alkoxy, sulphanyl, alkenyl, alkynyl, carbocyclyl or heterocyclyl is optionally further substituted with 0-6 groups selected from F, Cl, Br, I, hydroxyl, amino, carboxyl, C₁₋₆ alkyl, 3- to 6-membered carbocyclyl or 3- to 6-membered heterocyclyl, and the heterocyclyl contains 1-2 heteroatoms selected from N, O or S; n is selected from 1, 2 or 3;
the pharmaceutically acceptable salt is selected from benzenesulphonate, p-toluenesulphonate or methanesulphonate.

2. The use according to claim 1, **characterized in that** the compound represented by formula (I) is selected from one of the following structures: 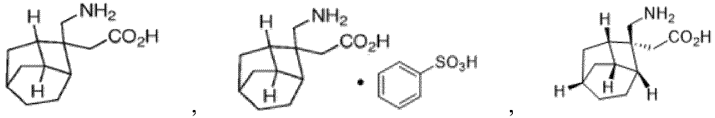 or

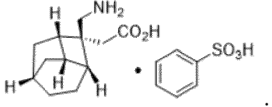

3. The use according to claim 1 or 2, **characterized in that** the central neuropathic pain comprises post-stroke pain, spinal cord injury pain, Parkinson's disease-related pain, and multiple sclerosis-related pain.

4. The use according to any one of claims 1-3, **characterized in that** the effective amount is based on a free base of formula (I), and the effective amount is 1-100 mg/day, preferably 5-20 mg/day.

5. The use according to claim 4, **characterized in that** the compound represented by formula (I), or the stereoisomer, the tautomer, the deuterated form, the solvate, the prodrug, the metabolite, the pharmaceutically acceptable salt, the cocrystal or the composition thereof is administered via a route selected from injection, drip, transdermal route, sublingual route, parenteral route, intraperitoneal route, rectal route, buccal route, nasal drip, inhalation, local delivery, subcutaneous route, intra-adipose route, intra-articular route, intraperitoneal route or intrathecal route.

6. The use according to claim 5, **characterized in that** the compound represented by formula (I), or the stereoisomer, the tautomer, the deuterated form, the solvate, the prodrug, the metabolite, the pharmaceutically acceptable salt, the cocrystal or the composition thereof is administered via a route selected from intravenous injection, intravenous drip, intra-arterial injection, intramuscular injection, subcutaneous injection, intra-articular injection, intraperitoneal injection, intrathecal injection or nasal drip.
